# EUROPEAN PATENT SPECIFICATION

(11) **EP 1 753 382 B1**
(45) Date of publication and mention of the grant of the patent: **21.01.2015**
(21) Application number: 04736713.1
(22) Date of filing: 11.06.2004
(51) Int. Cl.: A61F 13/15, A61L 15/46, A61K 9/50, A61F 13/551

(54) **AN ABSORBENT ARTICLE CONTAINING FRAGRANCE**
SAUGFÄHIGER ARTIKEL MIT EINEM DUFTSTOFF
ARTICLE ABSORBANT A PARFUM

(43) Date of publication of application: 21.02.2007
(73) Proprietor: SCA Hygiene Products AB, 405 03 Göteborg (SE)
(72) Inventor: ALBINO, Robert, S-435 43 Pixbo (SE)
(74) Representative: Andersson, Per Rune
(86) International application number: PCT/SE2004/000910
(87) International publication number: WO 2005/120412

(56) References cited:
- WO-A1-99/53967
- GB-A- 2 311 727
- US-A- 4 606 956
- US-A- 5 282 572
- US-A- 5 290 268
- US-A- 5 591 146
- US-A- 5 769 832
- US-A- 6 162 457

## Description

### TECHNICAL FIELD

The present invention relates to a fragrance means for an absorbent article such as a sanitary towel, a panty liner, a diaper or an incontinence pad. The invention also comprises a packaging containing at least one absorbent article and also a fragrance means.

### BACKGROUND ART

The handling and use of absorbent articles such as diapers, incontinence pads, sanitary towels and panty liners intended for the absorption of body fluids can often feel unhygienic on account of the rather unpleasant odour a used absorbent article can give off. Bad odour can also be embarrassing for the wearer when the article is being used. In order to increase the impression of cleanness and freshness and in order to counteract bad odour in absorbent articles of this kind, it has therefore been proposed that these be provided with additives in the form of various types of deodorants and aromatic substances, for example perfumes. However, some users of absorbent hygiene articles do not want the article to smell of perfume. Moreover, certain people are hypersensitive or allergic to perfumes and for this reason wish to avoid products with such additives.

It is not appropriate either for the article to emit a perfume fragrance when it is stored, for example in a handbag or in a bathroom cabinet. Fragrances which are a pleasant and fresh experience in one situation can appear less suitable in another. For example, it is perhaps not always appreciated if the entire contents of a handbag smell of perfume. Correspondingly, it may of course be appropriate not to release perfume fragrance uncontrolledly in storage spaces such as storerooms, shops and bathrooms. It is moreover a problem with aromatic substances that they fade over time and that they age and change fragrance when they are exposed to the environment.

US 5,769,833 describes a diaper with perfume zones arranged on the outside of a backing layer. The perfume zones comprise a binder with perfume-emitting means and are covered by detachable strips. When the strips are torn off, the aromatic substances in the perfume zones are released. The strips can be torn off at different times, for example in connection with the article being put on, while the article is being worn, or when the article is taken off after use.

Although it is advantageous that the user can activate the perfume zones personally if and when desired, the disposal of the detachable strips involves a problem for the user. Furthermore, there is a risk of the perfume zones being activated unintentionally if the strips are removed by mistake by becoming caught on another object. For example, the strips may already be torn off by accident when the article is taken out of its packaging, or by, during use, catching on a garment worn outside the absorbent article.

WO 93/09818 also describes an absorbent article such as a dressing or a sanitary towel with a fragrance-emitting area which is activated by detaching a separate protective layer.

One object of the present invention is to provide an absorbent article which emits perfume fragrance only if and when this is desirable.

Another object is to offer a possibility of providing an absorbent article with a means for fragrance emission.

### DISCLOSURE OF INVENTION

By way of the present invention, a fragrance means for an absorbent article of the kind referred to in the introduction and also a packaging containing an absorbent article and a fragrance means have been produced.

In this connection, a fragrance means made according to the invention is characterized mainly in that it comprises an activatable fragrance arranged on a carrier, by virtue of which the fragrance means has an active, fragrant state and an inactive, non-fragrant state. According to a preferred embodiment, the carrier comprises an attachment element for fastening the fragrance means to the absorbent article. Alternatively, the carrier can be arranged to be attached to an attachment element arranged on the absorbent article.

By way of the invention, it therefore possible to choose to provide an absorbent article with a fragrance means. The fragrance means is suitably supplied together with the absorbent article, or sold in separate packagings. The fragrance means can be of such a nature that it is applied to the absorbent article in its inactive form or can be designed for activation already before fastening to the absorbent article.

When the fragrance means is arranged to be activated after fastening to the absorbent article, this can of course be done at any time in connection with and/or during use of the absorbent article. For example, the fragrance means can be fully or partly activated before the article is placed adjacent to the body of the user or in connection with the removal of a used article. It is also possible to activate the fragrance means during use, that is to say when the article is being worn adjacent to the body.

The fragrance can be arranged in the attachment element of the fragrance means and can, for example, be incorporated in an adhesive attachment element or be applied to a touch and close surface.

An fragrance which is not protected in another way before activation, such as by encapsulation, is advantageously covered by a detachable protective layer which preserves the fragrance means in the inactive state. It may also be suitable to protect encapsulated fragrances with a protective layer in order to avoid unintentional activation by the capsules being broken by mistake. For fragrances which are activated by exposure when the protective layer is removed, the protective layer must of course be fragrance-tight. In this connection, suitable protective layers are siliconized paper, plastic film or similar impermeable materials. For fragrances which are activated mechanically, for example by tearing fragrance capsules apart when the protective layer is freed, or by rubbing or scraping, permeable protective layers, made of non-woven material for example, can be used.

One possibility for activating the fragrance means so that it is transferred from the inactive to the active state can therefore be by removal of the protective layer.

Suitable fragrances are those associated with cleanness and freshness and include various types of perfume fragrance, lavender fragrance, "washing detergent fragrance" and the like.

As mentioned above, the attachment element of the fragrance means can be an adhesive attachment element. The attachment element is advantageously covered by a detachable protective layer which is removed when the fragrance means is to be applied to an absorbent article. Mechanical attachment elements, such as hook and loop surfaces, can also be covered by detachable protective layers. If the fragrance means is provided with a hook and loop surface, this can either be arranged to interact with a corresponding specially arranged hook and loop attachment surface on the absorbent article or be designed so that it can be attached directly to the surface material of the article. In the latter case, the hook and loop surface on the fragrance means is suitably provided with hook elements which can hook onto a porous surface on the article, for example the liquid-permeable surface layer.

The carrier of the fragrance means can comprise a porous, fragrance-permeable material. Such an embodiment is particularly suitable if the fragrance is arranged in the attachment element of the fragrance means or in other embodiments where the fragrance is arranged inside the carrier or on a surface of the carrier which faces away from the environment when the fragrance means is applied to an absorbent article. When the carrier is fragrance-permeable, it is generally necessary to arrange a protective layer over the carrier in order to prevent the fragrance being released into the environment before this is desired.

It is also possible to arrange fragrances in a carrier in the form of a gel. In connection with hygiene articles, it is usually desirable to keep the fragrance means separate from the skin of the user. However, the scope of the invention also includes embodiments where fragrance is transferred to the skin of the user, for example by contact with a fragrance-carrying gel or an exposed oil or other fragrance-carrying liquid.

It may be suitable if the carrier is permeable to the fragrance in the fragrance means, for example by virtue of being porous or perforated. A fragrance-permeable carrier can be used, for example, when the fragrance is arranged in the fastening element or the fragrance is arranged between two layers in a laminate. In order for it to be possible for the fragrance to escape to the environment, it is necessary in such an embodiment that the carrier is capable of allowing the fragrance to pass through.

When the fragrance is protected by a detachable protective layer, this can be divided into two or more individually detachable parts. In such an embodiment, it is possible to activate parts of the fragrance at different times, for example when the absorbent article is put on, during use or before disposal. Different parts/surfaces of the fragrance means can have different fragrances, so that the user can choose a desired fragrance.

The carrier of the fragrance means can be any suitable material. The carrier can therefore comprise porous materials such as non-woven materials, tissue, paper, perforated plastic film or foamed materials. Alternatively, the carrier can be in the form of a plastic film or a similar impermeable material.

The fragrance can be arranged inside the carrier, for example by being impregnated in a porous layer, or on a surface of the carrier. The fragrance can also be arranged between two material layers, at least one of which is fragrance-permeable so that the fragrance can pass out to the environment. If the carrier includes further layers, all such layers which are not intended to be removed and are arranged to be located between the fragrance means and the environment when the fragrance means is attached to an absorbent article must be fragrance-permeable.

According to one embodiment of the invention, the fragrance is arranged in microcapsules, the fragrance means being transferable into the active state by mechanical action. Such mechanical action can be rubbing or scraping but can also consist in the capsules being broken apart in connection with the removal of a protective layer or separate activation layer.

It is also possible to use fragrances which are activated by heat or a combination of heat and mechanical action, for example by virtue of the fragrance being arranged in a carrier which softens and breaks when it is exposed to body heat and the forces which arise when an absorbent article is used. Another possibility is moisture-activated fragrances.

According to another embodiment of the invention, the fragrance in the inactive state is covered by a scrape-off, fragrance-tight coating. When the coating is removed, the fragrance means, which may be a fragrant oil arranged on a porous carrier for example, is exposed. As in the embodiment where the fragrance is covered by a divided detachable protective layer, or where the fragrance is encapsulated in microcapsules, it is possible in this embodiment to choose to activate the fragrance means only partly. It is also possible to activate different parts of the fragrance means at different times. Different parts of the fragrance can also have different fragrances, so that the user is offered a choice.

The invention also comprises a packaging containing at least one absorbent article, such as a sanitary towel, a panty liner, a diaper or an incontinence pad. The packaging is characterized in that it comprises at least one fragrance means of the kind described above.

Absorbent articles such as sanitary towels, incontinence pads for mildly incontinent people and panty liners are usually attached inside the briefs of the user by means of an adhesive attachment element. Such attachment elements are found in the form of self-adhesive glued surfaces which may cover all or parts of the outside of the liquid-impermeable surface layer. It is also common to use other types of attachment element such as friction surfaces, hook and loop surfaces and clips.

According to one embodiment of the invention, the absorbent article is of the kind which has an adhesive attachment element, the adhesive attachment element of the article being covered before use of the article by a detachable protective layer. The fragrance means can then in the inactive state be attached detachably to the detachable protective layer of the absorbent article. The attachment element of the fragrance means is suitably used for such fastening, the protective layer of the article serving a dual purpose as a protective layer for both the fastening element on the article and the fastening element on the fragrance means. In order to make it easier to detach the fragrance means from the protective layer, this is advantageously treated with release agent on the surface where the fragrance means is applied.

Correspondingly, the fragrance means in the inactive state can be attached detachably to the packaging. The fragrance means can then be attached either on the inside of the packaging or on its outside. If the packaging is a multi-pack, that is to say a packaging for more than one absorbent article, it may be suitable to arrange the fragrance means on the outside of the packaging, which increases the accessibility of the fragrance means when several articles remain in the packaging. For individually packaged articles, it may be more suitable if the fragrance means is arranged inside the packaging so that it becomes accessible at the same time as the absorbent article. The fragrance means does not have to be attached to the packaging but can be packed loosely with it.

In order to increase the choice for the user, the packaging can contain at least two fragrance means with fragrances which are different from one another or fragrance means with two or more fragrances on different parts/surfaces of the fragrance means.

It may also be advantageous if the article has a marking for suitable positioning of the fragrance means. Such a marking can be in the form of a decorative pattern, a differing colour, a differing material, text, a logo or the like. It is also possible to use touch-detectable indicators such as embossed surfaces, differing material or the like. Such an embodiment facilitates positioning and activation of the fragrance means when the absorbent article is being worn adjacent to the body.

### DESCRIPTION OF FIGURES

The invention will be described in greater detail below with reference to the figures shown in accompanying drawings, in which:
- Figure 1: shows a fragrance means according to the invention;
- Figures 2a-2d: show a section through variants of the fragrance means shown in Figure 1;
- Figure 3: shows a sanitary towel seen from the side which is intended to face away from the user during use, and a fragrance means according to the invention;
- Figure 4: shows the sanitary towel in Figure 4 with the fragrance means attached to the sanitary towel;
- Figure 5: shows a packaging wrapper with a sanitary towel and a fragrance means according to the invention;
- Figure 6: shows the packaging wrapper in Figure 5 folded up into an individual packaging containing the sanitary towel and the fragrance means;
- Figure 7: shows a packaging containing a number of absorbent articles and fragrance means packed with it, and
- Figure 8: shows a fragrance means according to another embodiment of the invention.

### DESCRIPTION OF ILLUSTRATIVE EMBODIMENTS

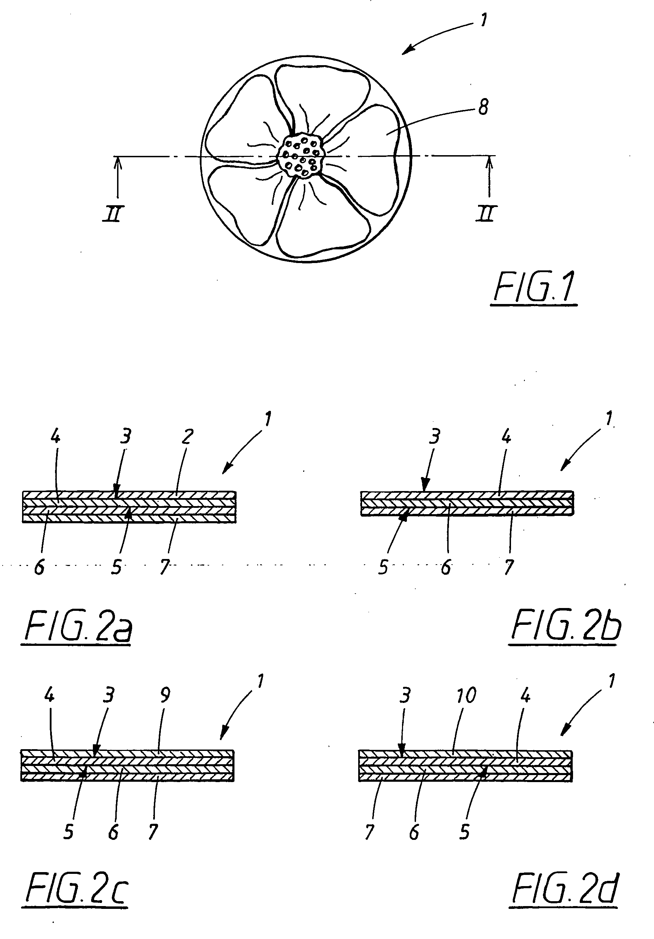

The fragrance means 1 shown in Figure 1 is shown from the surface which is intended to face outwards and which constitutes the fragrance-emitting surface when the fragrance means is attached to an absorbent article. The fragrance means can advantageously be provided with some form of decoration or symbol, for example a flower 8 as shown in Figure 1. Such a symbol or decoration can be used to provide information about the type of fragrance the fragrance means 1 contains and to show which side of the fragrance means is fragrance-emitting.

In accordance with the invention, the fragrance means can be constructed in various different ways, a number of which are exemplified by the cross-sectional illustrations in Figures 2a-2d. Figure 2a shows a construction where the uppermost layer in the figure is a first protective layer 2 which is attached detachably to a first surface 3 of a carrier 4 in the form of a material layer containing a fragrance. Arranged on the opposite, second surface 5 of the carrier 4 is a layer 6 of self-adhesive glue, which is in turn covered by a second protective layer 7.

The carrier layer 4 can be a porous material such as a non-woven layer, a net, a perforated plastic film, a woven fabric, a tissue layer, a paper layer, a foam layer or the like and can have the fragrance arranged as a coating on one or both of the surfaces 3, 5 or be impregnated with the fragrance. Alternatively, the carrier layer 4 can be a fragrance-tight material such as a plastic film, the fragrance being arranged as a coating on the first surface 3 of the carrier layer 4.

The adhesive layer 6 is suitably a conventional self-adhesive adhesive, for example a hot melt adhesive, and the protective layer 7 which is arranged over the adhesive layer 6 is then suitably a conventional release-agent-treated protective layer, such as a siliconized paper layer. It is not necessary for the adhesive layer 6 to cover the entire second surface 5 of the carrier layer 3. For example, the adhesive layer 6 can be arranged in a discontinuous coating. It may also be advantageous to leave at least part of the area next to the edge of the fragrance means 1 free of glue, so that removal of the protective layer 7 is facilitated.

The protective layer 2 which covers the first surface 4 of the carrier layer 3 is suitably a fragrance-tight plastic layer. Such a plastic layer can be held in place electrostatically or by an adhesive which is easily freed from the carrier layer 3. If, as shown in Figure 1, the fragrance means is provided with a decoration 8, this can be printed on the protective layer 2. Alternatively, the protective layer 2 can be transparent, so that a decorative image can be seen through the layer.

Figure 2b shows another embodiment of a fragrance means 1 according to the invention. In this case, the fragrance means 1 comprises a porous, fragrance-permeable carrier 4 with a first surface 3 which is intended to face outwards towards the environment when the fragrance means 1 is attached to an absorbent article and with a second surface 5 which is provided with a coating 6 of self-adhesive adhesive. The adhesive layer 6 contains a mechanically activatable fragrance encapsulated in breakable microcapsules. The adhesive layer 6 is also covered by a detachable protective layer 7. The fragrance means 1 can be arranged so that it is activated by virtue of the microcapsules breaking when the protective layer 7 is removed in order to expose the adhesive layer 6 for fastening the fragrance means 1 to an absorbent article. Alternatively, the fragrance means can be activated by virtue of the microcapsules being crushed in connection with the fragrance means 1 being fastened to an absorbent article. When the microcapsules are broken, the fragrance is released into the environment through the fragrance-permeable carrier 4.

According to an alternative embodiment, the carrier 4 is fragrance-tight. A fragrance means 1 according to such an embodiment is suitable for fastening to a liquid-permeable layer on an absorbent article, the fragrance being released into the absorbent article, from where it can diffuse further. In such an embodiment, the fragrance-tight carrier 4 prevents the fragrance coming into direct contact with the skin of the wearer when the article is being worn.

The carrier 4 can also be a laminate consisting of two material plies with a fragrance located between the material plies. In such an embodiment, at least one material ply must be fragrance-permeable.

Figure 2c shows a fragrance means 1 with a scrape-off upper coating 9 which protects a first surface 3 of a carrier 4 containing a fragrance. The fragrance means 1 also has an adhesive layer 6 arranged on the second surface 5 of the carrier 4. The adhesive layer is covered by a protective layers 7.

The fragrance means 1 shown in Figure 2c is activated by the upper coating 9 being scraped off. This can be effected at any time, either before the fragrance means 1 is attached to an absorbent article or after the fragrance means has been attached to an absorbent article. It is also possible to expose and thus activate the fragrance only partly. The fragrance means 1 in Figure 2c can therefore be used repeatedly for the release of fragrance. The user can therefore choose to activate all or parts of the fragrance means at different times, according to personal choice. For users who do not wish to wear fragrance means close to the body, it may nevertheless be desirable for it to be possible to activate the fragrance means in connection with the absorbent article being disposed of so as to avoid bad odour of the used article.

Figure 2d shows a fragrance means with an upper fragrance layer 10 with fragrance encapsulated in breakable microcapsules. The fragrance layer 10 is applied to a first surface 3 of a carrier 4, and an adhesive layer 6 is applied to the second surface 5 of the carrier 4. The adhesive layer 6 is covered by a removable protective layer 7.

The fragrance means 1 is activated by the fragrance layer 10 being rubbed or scraped so that the microcapsules are broken and the fragrance is released. As in the embodiment in Figure 2c, it is possible to activate the fragrance means only partly and at different times. Such a possibility can also be brought about for the embodiment shown in Figure 2a by the upper protective layer 2 being designed in two or more parts which can be removed separately.

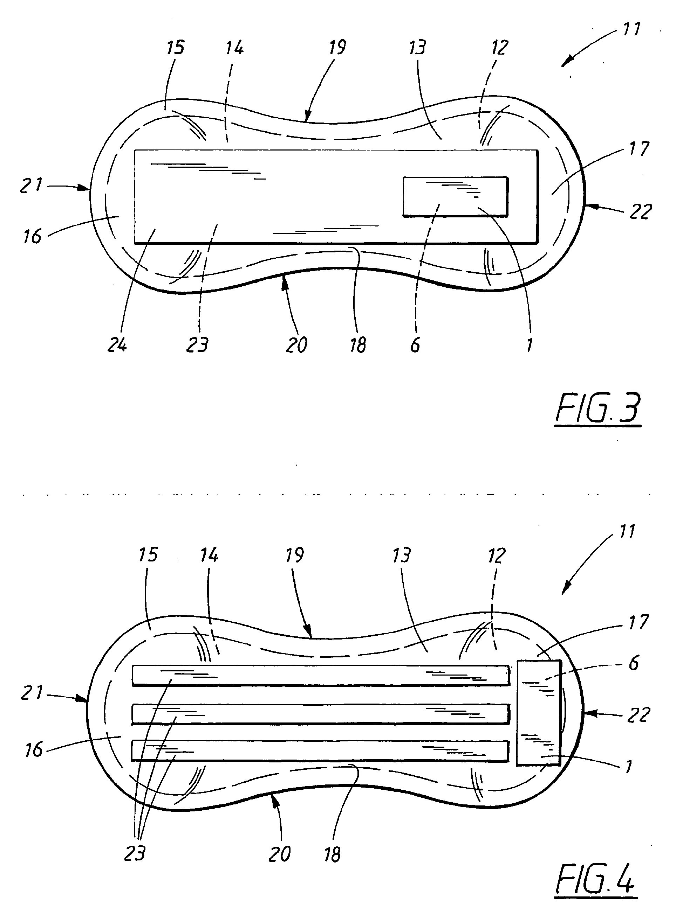

Figures 3 and 4 show a sanitary towel 11. The sanitary towel 11 is shown from the side which is intended to face the underwear of the user when the towel is being worn. The sanitary towel 11 comprises a liquid-permeable surface layer 12, arranged on that side of the sanitary towel 11 which is intended to face the user during use, a liquid-impermeable surface layer 13, arranged on that side of the sanitary towel 11 which is intended to face away from the user-during use, and an absorbent body 14 enclosed between the two surface layers 12, 13.

The material of the liquid-permeable surface layer 12 can be, for example, a perforated plastic film, a net made of plastic or textile material, a non-woven material or a laminate consisting of, for example, a perforated plastic layer and a non-woven layer. The plastic is usually a thermoplastic, such as polyethylene or polypropylene. Suitable non-woven materials can be made of natural fibres, such as cellulose or cotton, or of synthetic fibres, such as polyethylene, polypropylene, polyester, polyurethane, nylon or regenerated cellulose. It is of course also possible to use non-woven materials made from fibre mixtures.

It is not necessary for the liquid-permeable surface layer 12 actually to consist of a separate material layer. For example, the liquid-permeable covering layer can constitute an integrated part of the absorbent body 14. Absorbent foamed materials, for example, often have a sufficiently good cohesive capacity that a separate liquid-permeable surface layer can be dispensed with. Use can also be made of an absorbent non-woven material, which can be included as a component in an absorbent body and at the same time constitute a liquid-permeable covering layer. Such constructions are not uncommon in very thin towels and panty liners.

The liquid-impermeable covering layer 13 consists of a liquid-impermeable material. Thin, liquidtight plastic films are suitable for the purpose. However, it is also possible to use materials which are originally liquid-permeable but have been provided with a coating of plastic, resin or other sealing material In this way, leakage of liquid from the underside of the absorbent article is prevented. The liquid-impermeable covering layer 13 can therefore consist of any material which is skin-friendly and meets the liquid-impermeability criterion. Examples of materials suitable as a barrier layer are plastic films, non-woven materials and various types of laminate. Plastic films which can be used are, for example, those made of polyethylene, polypropylene or polyester. Alternatively, the liquid-impermeable covering layer 13 can consist of a laminate made of a liquid-impermeable plastic layer facing the absorbent body 14 and a non-woven layer facing the underwear of the user. Such a construction provides a leakproof barrier layer with a textile feel.

In the same way as with the liquid-permeable covering layer 12, it is not necessary for the liquid-impermeable covering layer 13 to consist of a separate layer. It is therefore possible to imagine the liquid-impermeable covering layer 13 constituting an integrated part of an absorbent material, for example an absorbent foam layer with a liquid-impermeable surface.

The absorbent body 14 can advantageously be made mainly from cellulose fluff pulp. This can be in the form of rolls, bales or sheets which are dry-defibred and converted in fluffed form into a pulp mat, with or without the addition of what are known as superabsorbents, which are polymers with a capacity for absorbing several times their own weight of water or body fluid. Examples of other materials which can be used are various types of natural fibre such as cotton fibres, peat or the like. It is of course also possible to use absorbent synthetic fibres or mixtures of natural fibres and synthetic fibres. The absorbent material can also contain other components, such as liquid-spreading means or binders such as, for example, thermoplastic fibres which have been heat-treated so as to hold short fibres and particles together to form a cohesive unit. It is also possible to use various types of absorbent foamed material in the absorbent body 14.

The two covering layers 12, 13 are interconnected outside the absorbent body 14 and form a projecting edge 15 around the entire periphery of the sanitary towel. The covering layers can be joined together in any suitable way, such as by adhesive, sewing or welding using heat or ultrasound.

The sanitary towel 11 is essentially hourglass-shaped and has two end portions 16, 17 and an intermediate narrower crotch portion 18 intended to be arranged between the legs of the user. The sanitary towel 11 also has two inwardly curved side edges 19, 20 and two outwardly curved end edges 21, 22.

The division of the sanitary towel 11 into two end portions 16, 17 and a crotch portion 18 is not to be understood as there being well-defined boundaries between the different portions 16, 17, 18 but is primarily intended to facilitate the description of the sanitary towel on the basis of the differences which exist between the different portions depending on how they are intended to be positioned in relation to the body of a user. The transition between the different portions 16-18 does not therefore take place at defined transverse lines but rather within transition areas located at a distance of approximately one third of the length of the sanitary towel 11 from the end edges 21, 22 of the sanitary towel. The crotch portion 18 constitutes that part of the sanitary towel 11 which is intended to receive and absorb the bulk of the liquid discharged into the sanitary towel during use.

The sanitary towel 11 is provided with an adhesive fastening element 23 in the form of three strands of adhesive which are attached in the crotch portion of the briefs of the user when the towel 11 is worn. Before use, the fastening element 23 is, as shown in Figure 3, protected in a conventional way by a detachable protective strip 24 which is removed when the adhesive is to be exposed for fastening. The fastening element 23 shown is only an example of the design of an adhesive fastening element. Other patterns and positionings of fastening element are of course possible.

The sanitary towel 11 in Figure 3 also has a fragrance means 1 fastened detachably to the protective strip 24 of the fastening element 23. The fragrance means 1 can be designed in any of the ways shown in Figures 2a-2d. The fragrance means 1 can therefore contain fragrance which is enclosed in microcapsules which break when they are subjected to rubbing, scraping or other mechanical action, or fragrance can be applied to or in a carrier and be covered by a removable protective layer or a scrape-off coating. The fragrance means is therefore, as shown in Figure 3, present in an inactive, non-fragrant form which it retains until the user chooses to activate the fragrance means 1.

The attachment element 6, for example an adhesive glue layer, of the fragrance means 1 is suitably used in order to attach the fragrance means to the protective strip 24 of the sanitary towel. The protective strip 24 is therefore used as a protective layer for both the fastening element 23 on the sanitary towel and the fastening element 6 on the fragrance means 1. In order to make it easier to detach the fragrance means 1 from the protective strip 24, the latter is advantageously treated with release agent on the surface to which the fragrance means 1 is applied.

If the attachment element 6 on the fragrance means 1 is a hook and loop surface provided with hooks, the protective strip 24 can suitably have a corresponding loop surface or be designed with a layer of non-woven to which the touch and close surface provided with hooks can be attached.

Figure 4 shows the sanitary towel 11 after the protective strip 24 has been removed so that the fastening element 23 has become visible. The fragrance means 1 has also been detached from the protective strip 24 and attached to the liquid-impermeable covering layer 13 in one end portion 17 of the sanitary towel 11. The positioning shown is only an example of where the fragrance means 1 can be applied. It is alternatively possible to apply the fragrance means 1 over the attachment element 23 of the sanitary towel 11. As described above, moreover, certain types of fragrance means 1 are suited for fastening to the liquid-permeable covering layer 12.

The sanitary towel 11 in Figures 3 and 4 can of course be provided with more than one fragrance means 1. Such further fragrance means 1 can be used for locating fragrance in different places on the sanitary towel. It is also possible to apply fragrance means with different fragrances to the protective strip 24, so that the user can select a desired fragrance.

Positioning the fragrance means 1 in one of the end portions 16, 17 of the sanitary towel makes it possible to reach the fragrance means 1 easily for activation both before the sanitary towel is fitted in a pair of briefs and during use and after use when the sanitary towel is to be disposed of. The positioning shown does not interfere with the fastening element 23 either, but the user can reach the fragrance means without risking becoming stuck on the fastening element 23.

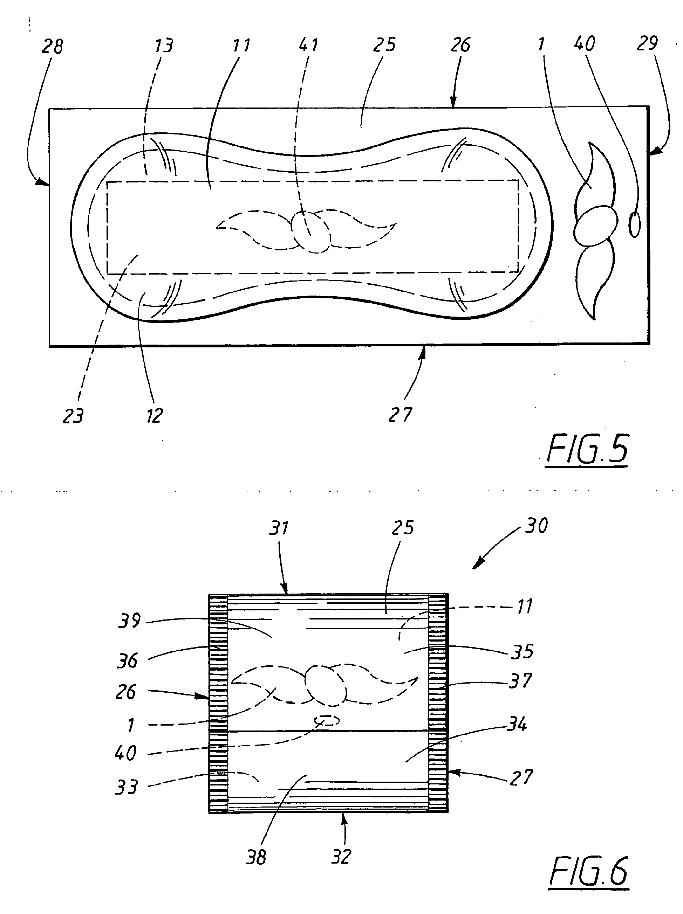

Figures 5 and 6 show a sanitary towel 11 and a packaging wrapper 25. The packaging wrapper 25 consists of a rectangular piece of flexible sheet material, such as a plastic film, a paper sheet, a non-woven material or a material laminate. The packaging wrapper 25 has two long sides 26, 27 and two short sides 28, 29.

Figure 5 shows the packaging wrapper 25 before it is folded together to form a packaging. The sanitary towel 11 has essentially the same construction as the sanitary towel 11 shown in Figures 3 and 4 and is positioned on the packaging wrapper 25 with the liquid-impermeable covering layer 13 facing the packaging wrapper 25 and with the liquid-permeable covering layer 12 facing away from the packaging wrapper 25. An absorbent body 14 is arranged between the covering layers 12, 13. The sanitary towel 11 is also provided with an adhesive fastening element 23 for fastening the sanitary towel 11 in a pair of briefs. Figure 5 shows the sanitary towel 11 with the adhesive fastening element 23 temporarily attached to the inside of the packaging wrapper 25, that is to say that side of the packaging wrapper which faces the liquid-impermeable covering layer 13 of the packaged sanitary towel 11. The opposite side of the packaging wrapper 25 therefore constitutes its outside. In order to make it easier to detach the sanitary towel 11 from the packaging wrapper 25, the packaging wrapper 25 is suitably treated with release agent, for example with a silicone coating applied within the area where the fastening element 23 is attached. By virtue of the sanitary towel 11 being temporarily attached to the packaging wrapper 25, the packaging wrapper 25 serves as a protective layer for the fastening element 23 of the sanitary towel 11 until the sanitary towel is to be used for absorption of body fluid. Alternatively, it is of course possible to provide the fastening element 23 with a separate protective layer, as shown in Figure 3.

Figure 6 shows the packaging wrapper 25 folded together to form a packaging 30 enclosing the sanitary towel 11. In the example shown, the sanitary towel 11 and the packaging 25 are folded up together, but it is of course possible to fold them up individually. Such a procedure is especially suitable when the sanitary towel 11 is provided with a separate protective layer for the fastening element 23.

The sanitary towel 11 and the packaging wrapper 25 are folded along two folding lines 31, 32 which divide the packaging wrapper into a first end portion 33, an intermediate portion 34 and a second end portion 35. The first end portion 33 and the intermediate portion 34 are joined at side joins 36, 37 along the long sides 26, 27 of the packaging wrapper 25 and in this way form a container part 38. The second end portion 35 is folded down over the outside of the intermediate portion 34 and forms a cover part 39 which closes the packaging 30. In order to keep the packaging 30 closed until the sanitary towel 11 is to be taken out of the packaging 30 and made available for use as an insert in a pair of briefs, the cover part 39 is attached to the outside of the container part 38 on the one hand by being attached at the side joins 36, 37 and on the other hand by way of an adhesive attachment means 40 arranged on the inside of the cover part 39. When the packaging 30 is closed, as shown in Figure 6, the attachment means 40 is located between the inside of the cover part 39 and the outside of the container part 38 and connects these two surfaces.

The side joins 36, 37 are suitably of the kind which can be torn open without the packaging material breaking. Such joins can be brought about by means of adhesive or by thermal welding, usually in combination with embossing. In order to make the joins breakable, it may be necessary to treat the packaging material. For example, it may be appropriate to treat parts of the material with release agent when the joins are adhesive joins. In the case of welded joins, use is often made of lacquer or similar polymer coatings in order to prevent material layers included in the join being fused.

When the packaging is opened, the breakability of the side joins 36, 37 means that the packaging wrapper 25 can be unfolded completely into the plane state shown in Figure 5, which makes it considerably easier to detach the sanitary towel 11 from the packaging wrapper 25. When packaging absorbent articles which are not attached to the packaging wrapper 25, it is not necessary to provide the packaging 30 with breakable side joins 36, 37. On the other hand, it is appropriate for the cover part 39 to be attached detachably to the container part 38 at the side joins 36, 37 in order for it to be possible for the packaging 30 to be opened without the packaging material being torn apart.

A fragrance means 1 in accordance with the invention is attached on the inside of the packaging wrapper 25, inside one short side 29, close to the adhesive attachment means 40 on the cover part 35. The fragrance means is attached detachably to the packaging wrapper 25, for example by means of the attachment element of the fragrance means. If the attachment element is of a kind which does not attach to the packaging wrapper, however, it may be suitable alternatively to attach that surface of the fragrance means 1 opposite the attachment element to the packaging wrapper 25. In such an embodiment, the fragrance means in Figure 5 is therefore turned with its attachment element towards the observer of the figure.

The sanitary towel 11 in Figure 5 also has a marking 41 which imitates the shape of the fragrance means 1. The marking is intended to indicate visually for the user where the fragrance means 1 is intended to be located. As an alternative to the marking shown, a different pattern, text or simply a differing colour can of course be used. Visually identifiable markings can of course be used in order to indicate suitable positioning of the fragrance means 1 for any of the embodiments described herein.

In the examples described, the absorbent article is a sanitary towel. However, the invention is applicable to similar absorbent articles such as diapers, incontinence pads, panty liners or the like.

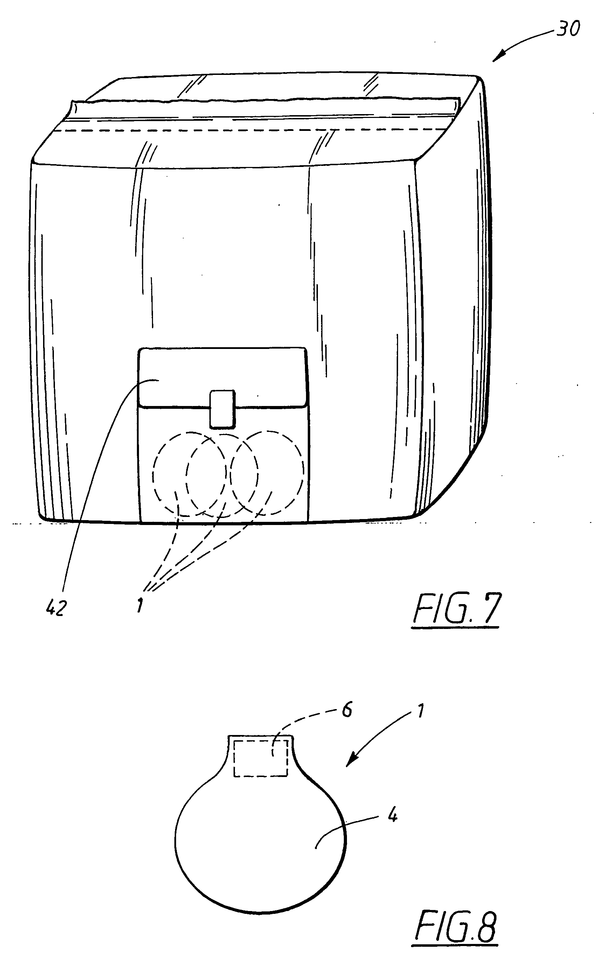

Figure 7 shows a packaging 30 which accommodates a number of absorbent articles (not shown). The packaging 30 is provided with an openable and reclosable pocket 42 on the outside of the packaging 30, into which pocket 42 a number of fragrance means 1 in accordance with the invention are put. The number of fragrance means 1 suitably corresponds to the number of absorbent articles in the packaging, so that it is possible to apply a fragrance means 1 to each of the absorbent articles as they are used. The positioning of the fragrance means 1 on the outside of the packaging makes it possible to reach a fragrance means 1 easily, even when no or only a few articles have been taken out of the packaging 30.

As an alternative to the storage pocket 42 shown in Figure 7, the fragrance means 1 can be arranged in the form of detachable labels on the inside or outside of the packaging. Another alternative is of course quite simply to include a number of loose fragrance means 1 inside the packaging.

Figure 8 shows another fragrance means 1 according to the invention. The fragrance means 1 shown in Figure 8 comprises a carrier 4 consisting of a porous material layer. The carrier is provided with an attachment means 6 in the form of an adhesive surface or a hook and loop surface which occupies only a small part of the surface of the carrier and is positioned at one end of the carrier 4. The carrier 4 contains a mechanically activatable fragrance, for example in the form of a fragrance enclosed in microcapsules. When the fragrance is to be activated, this is effected by that part of the fragrance means 1 free of attachment means being subjected to rubbing so that the fragrance is released.

## Claims

1. A packaging (30) containing at least one unused absorbent article (11), such as a sanitary towel, a panty liner, a diaper or an incontinence pad, **characterized in that** the packaging (30) comprises at least one fragrance means (1) for the absorbent article (11), said fragrance means (1) comprising an activatable fragrance arranged on a carrier (4), by virtue of which the fragrance means (1) has an active, fragrant state and an inactive, non-fragrant state, wherein the fragrance means (1) in its inactive form is covered by a scrape-off fragrance tight coating, and wherein the fragrance means can be activated by mechanical action such as scraping of the fragrance tight coating so that fragrance is emitted only if and when this is desirable.

2. A packaging (30) according to Claim 1, **characterized in that** the carrier (4) comprises an attachment element (6) for fastening the fragrance means (1) to the absorbent article (11).

3. A packaging (30) according to Claim 2, **characterized in that** the fragrance is arranged in the attachment element (6) of the fragrance means (1).

4. A packaging (30) according to any one of Claims 2-3, **characterized in that** the attachment element (6) is an adhesive attachment element.

5. A packaging (30) according to any one of claims 2-4, **characterized in that** the attachment element (6) is covered by a detachable protective layer (7).

6. A packaging (30) according to any one of the preceding claims, **characterized in that** the carrier (4) comprises a porous, fragrance-permeable material.

7. A packaging (30) according to Claim 6, **characterized in that** the carrier (4) comprises a non-woven material.

8. A packaging (30) according to Claim 6, **characterized in that** the carrier (4) comprises a perforated plastic layer.

9. A packaging (30) according to any one of the preceding claims, **characterized in that** the fragrance means is arranged between two material layers, at least one of which is fragrance-permeable.

10. A packaging (30) according to Claim 1, **characterized in that** the fragrance is arranged in microcapsules, the fragrance means (1) being transferable into the active state by mechanical action.

11. A packaging (30) according to anyone of the above claims, **characterized in that** the absorbent article (11) has an adhesive attachment element (23), the adhesive attachment element of the article being covered by a detachable protective layer (24), and **in that** the fragrance means (1) in the inactive state is attached detachably to the detachable protective layer (24) of the absorbent article (11).

12. A packaging (30) according to anyone of claims 1-11, **characterized in that** the fragrance means (1) in the inactive state is arranged detachably on the packaging (30).

13. A packaging (30) according to any one of the above claims, **characterized in that** the packaging (30) comprises at least two fragrance means (1) with fragrances which are different from one another.

14. A packaging (30) according to any one of the above claims, **characterized in that** the article (11) has a marking (42) for indicating suitable positioning of the fragrance means.

## Patentansprüche

1. Verpackung (30) enthaltend zumindest einen ungenutzten absorbierenden Artikel (11), wie beispielsweise eine Binde, eine Slipeinlage, eine Windel oder eine Inkontinenzeinlage, **dadurch gekennzeichnet, dass** die Verpackung (30) zumindest ein Duftmittel (1) für den absorbierenden Artikel (11) aufweist, welches Duftmittel (1) einen auf einem Träger (4) angeordneten aktivierbaren Duftstoff umfasst, aufgrund dessen das Duftmittel (1) einen aktiven, duftenden Zustand und einen inaktiven, nicht duftenden Zustand aufweist, wobei das Duftmittel (1) in seiner inaktiven Form von einer abkratzbaren, duftdichten Beschichtung überdeckt ist, und wobei das Duftmittel durch mechanische Einwirkung aktiviert werden kann, wie beispielsweise durch Abkratzen der duftdichten Beschichtung, so dass ein Duft nur ausgesendet wird, wenn dieses erwünscht ist.

2. Verpackung (30) nach Anspruch 1, **dadurch gekennzeichnet, dass** der Träger (4) ein Befestigungselement (6) zur Befestigung des Duftmittels (1) an dem absorbierenden Artikel (11) aufweist.

3. Verpackung (30) nach Anspruch 2, **dadurch gekennzeichnet, dass** das Duftmittel in dem Befestigungselement (6) des Duftmittels (1) angeordnet ist.

4. Verpackung (30) nach einem der Ansprüche 2-3, **dadurch gekennzeichnet, dass** das Befestigungselement (6) ein klebendes Befestigungselement ist.

5. Verpackung (30) nach einem der Ansprüche 2-4, **dadurch gekennzeichnet, dass** das Befestigungselement (6) von einer abnehmbaren Schutzschicht (7) überdeckt ist.

6. Verpackung (30) nach einem der vorgehenden Ansprüche, **dadurch gekennzeichnet, dass** der Träger (4) ein poröses, duftdurchlässiges Material umfasst.

7. Verpackung (30) nach Anspruch 6, **dadurch gekennzeichnet, dass** der Träger (4) ein Vliesmaterial umfasst.

8. Verpackung (30) nach Anspruch 6, **dadurch gekennzeichnet, dass** der Träger (4) eine perforierte Kunststoffschicht umfasst.

9. Verpackung (30) nach einem der vorgehenden Ansprüche, **dadurch gekennzeichnet, dass** das Duftmittel zwischen zwei Materialschichten angeordnet ist, von denen zumindest eine duftdurchlässig ist.

10. Verpackung (30) nach Anspruch 1, **dadurch gekennzeichnet, dass** das Duftmittel in Mikrokapseln angeordnet ist, wobei das Duftmittel (1) durch mechanische Einwirkung in den aktiven Zustand übertragbar ist.

11. Verpackung (30) nach einem der vorgehenden Ansprüche, **dadurch gekennzeichnet, dass** der absorbierende Artikel (11) ein klebendes Befestigungselement (23) aufweist, wobei das klebende Befestigungselement des Artikels von einer abnehmbaren Schutzschicht (24) überdeckt ist, und dass das Duftmittel (1) in dem inaktiven Zustand an der abnehmbaren Schutzschicht (24) des absorbierenden Artikels (11) lösbar befestigt ist.

12. Verpackung (30) nach einem der Ansprüche 1-11, **dadurch gekennzeichnet, dass** das Duftmittel (1) in dem inaktiven Zustand an der Verpackung (30) lösbar angeordnet ist.

13. Verpackung (30) nach einem der vorgehenden Ansprüche, **dadurch gekennzeichnet, dass** die Verpackung (30) zumindest zwei Duftmittel (1) mit voneinander unterschiedlichen Duften aufweist.

14. Verpackung (30) nach einem der vorgehenden Ansprüche, **dadurch gekennzeichnet, dass** der Artikel (11) eine Markierung (42) zur Anzeige des geeigneten Positionierens des Duftmittels aufweist.

## Revendications

1. Emballage (30) contenant au moins un article absorbant non utilisé (11), tel qu'une serviette hygiénique, un protège-slip, une couche ou une protection contre l'incontinence, **caractérisé en ce que** l'emballage (30) comprend au moins un moyen de parfum (1) pour l'article absorbant (11), le moyen de parfum (1) comprenant un parfum activable disposé sur un support (4), au moyen duquel le moyen de parfum (1) présente un état parfumé, actif, et un état inactif, non parfumé, le moyen de parfum (1) dans sa forme inactive étant recouvert par un revêtement étanche à enlèvement par raclage, et le moyen de parfum pouvant être activé par une action mécanique telle que raclage du revêtement étanche au parfum si bien que le parfum n'est émis que si et quand il est désirable.

2. Emballage (30) selon la revendication 1, **caractérisé en ce que** le support (4) comprend un élément de fixation (6) pour fixer le moyen de parfum (1) à l'article absorbant (11).

3. Emballage (30) selon la revendication 2, **caractérisé en ce que** le parfum est disposé dans l'élément de fixation (6) du moyen de parfum (1).

4. Emballage (30) selon l'une quelconque des revendications 2 à 3, **caractérisé en ce que** l'élément de fixation (6) est un élément de fixation adhésif.

5. Emballage (30) selon l'une quelconque des revendications 2 à 4, **caractérisé en ce que** l'élément de fixation (6) est recouvert par une couche protectrice détachable (7).

6. Emballage (30) selon l'une quelconque des revendications précédentes, **caractérisé en ce que** le support (4) comprend un matériau et poreux perméable à parfum.

7. Emballage (30) selon la revendication 6, **caractérisé en ce que** le support (4) comprend un matériau non-tissé.

8. Emballage (30) selon la revendication 6, **caractérisé en ce que** le support (4) comprend une couche de matière plastique perforée.

9. Emballage (30) selon l'une quelconque des revendications précédentes, **caractérisé en ce que** le moyen de parfum est disposé entre deux couches de matériau, dont au moins l'une est perméable à parfum.

10. Emballage (30) selon la revendication 1, **caractérisé en ce que** le parfum est disposé dans des microcapsules, le moyen de parfum (1) pouvant être transféré dans l'état actif par une action mécanique.

11. Emballage (30) selon l'une quelconque des revendications précédentes, **caractérisé en ce que** l'article absorbant (11) présente un élément de fixation adhésif (23), l'élément de fixation adhésif de l'article étant recouvert par une couche protectrice détachable (24), et **en ce que** le moyen de parfum (1) à l'état inactif est fixé de façon amovible à la couche protectrice détachable (24) de l'article absorbant (11).

12. Emballage (30) selon l'une quelconque des revendications 1 à 11, **caractérisé en ce que** le moyen de parfum (1) à l'état inactif est agencé de manière détachable sur l'emballage (30).

13. Emballage (30) selon l'une quelconque des revendications précédentes, **caractérisé en ce que** l'emballage (30) comprend au moins deux moyens de parfum (1) avec des parfums qui sont différents les uns des autres.

14. Emballage (30) selon l'une quelconque des revendications précédentes, **caractérisé en ce que** l'article (11) présente un marquage (42) pour indiquer le positionnement approprié des moyens de parfum.
